**Europäisches Patentamt**

**European Patent Office**

**Office européen des brevets**

(19)

(11) Veröffentlichungsnummer: **0 208 096**
**B1**

(12) # EUROPÄISCHE PATENTSCHRIFT

(45) Veröffentlichungstag der Patentschrift:
06.12.89

(51) Int. Cl.⁴: **G01N 25/08**, G01N 27/18,
G01N 33/28, B60T 17/22

(21) Anmeldenummer: 86106793.2

(22) Anmeldetag: 17.05.86

(54) Verfahren und Vorrichtung zur Ermittlung der Beschaffenheit und/oder des Zustandes einer hydraulischen Flüssigkeit.

(30) Priorität: 26.06.85 DE 3522774

(43) Veröffentlichungstag der Anmeldung:
14.01.87 Patentblatt 87/3

(45) Bekanntmachung des Hinweises auf die Patenterteilung:
06.12.89 Patentblatt 89/49

(84) Benannte Vertragsstaaten:
DE FR GB IT SE

(56) Entgegenhaltungen:
EP-A- 0 074 415
DE-A- 2 658 215
DE-A- 2 721 232
DE-A- 3 241 078
DE-A- 3 317 638

Die Akte enthält technische Angaben, die nach dem
Eingang der Anmeldung eingereicht wurden und die
nicht in dieser Patentschrift enthalten sind.

(73) Patentinhaber: ALFRED TEVES GmbH,
Guerickestrasse 7 Postfach 119 155, D-6000 Frankfurt
am Main(DE)

(72) Erfinder: Klein, Hans-Christof, Hofheimer Strasse 22,
D-6234 Hattersheim(DE)
Erfinder: Lohberg, Peter, Am Ringelsberg 7,
D-6382 Friedrichsdorf(DE)

(74) Vertreter: Blum, Klaus-Dieter, Dipl.-Ing., c/o ALFRED
TEVES GMBH Guerickestrasse 7,
D-6000 Frankfurt/Main 90(DE)

ACTORUM AG

## Beschreibung

Die Erfindung bezieht sich auf ein Verfahren zur Ermittlung der Beschaffenheit einer Druckübertragungsflüssigkeit, insbesondere zur Kontrolle oder Überwachung des Wassergehaltes einer hygroskopischen Bremsflüssigkeit, bei dem ein elektrisch beheizbares Sensorelement in die Flüssigkeit eingetaucht und mit konstantem elektrischen Strom beschränkt wird und bei dem der temperaturabhängige elektrische Widerstand des Sensorelementes gemessen wird. Vorrichtungen, mit denen sich ein solches Verfahren durchführen läßt, gehören ebenfalls zur Erfindung.

Es wurde bereits ein Verfahren zum Ermitteln der Siedetemperatur von hygroskopischen Flüssigkeiten, z.B. von Bremsflüssigkeiten, beschrieben (europäische Patentanmeldung EP-A 0 074 415), bei dem ein als Heiz- und Temperaturmeßelement dienender Körper in die zu kontrollierende Flüssigkeit eingetaucht wird. Das elektrische Aufheizen wird solange fortgesetzt, bis eine örtliche Verdampfung der Flüssigkeit einsetzt, was wiederum ein Konstantbleiben der Drahttemperatur infolge der Siedekühlung zur Folge haben soll. Diejenige Temperatur, bei der sich Dampfblasen bilden, wird als Maß für den Siedepunkt der Flüssigkeit gewertet.

Es ist zumindest fraglich, ob sich auf diese Weise tatsächlich eine Meßgröße gewinnen läßt, die Rückschlüsse auf die Siedetemperatur und auf den Wassergehalt der Bremsflüssigkeit zuläßt.

Die Heizstrecke eines derartigen Meß-Elementes mit einem konstanten Strom zu beschicken und den sich einstellenden, dem Widerstand der Heizstrecke proportionalen Spannungsabfall als Maß für den Siedepunkt auszuwerten, ist ebenfalls schon bekannt (deutsche Offenlegungsschrift 3 241 078).

Ferner ist durch die DE-OS 2 658 215, von der ausgehend die Oberbegriffe der vorliegenden Ansprüche 1 und 5 gebildet worden sind, eine Vorrichtung bekannt zur Messung des Siedepunktes einer Flüssigkeit oder des Punktes, bei welchem der Flüssigkeitsstrom durch Dampfblasenbildung unterbrochen wird. Es wird eine Probe der Flüssigkeit in einen aufheizbaren Behälter eingebracht. Der Behälter wird verschlossen und die Flüssigkeit aufgeheizt. Sobald sich Dampfblasen bilden, entleert sich der Behälter über einen Siphon, der vom Boden des Behälters nach außen führt. Über eine Elektrode, die von oben in den Behälter teilweise hineinragt, fließt elektrischer Strom, solange sich noch genügend Flüssigkeit in dem Behälter befindet. Ein Temperaturdetektor läßt erkennen, bei welcher Temperatur infolge der Dampfblasenbildung das Gefäß sich so weit entleert, daß der über die Elektrode fließende Strom unterbrochen wird.

Schließlich wurde auch schon eine Vorrichtung beschrieben, die im wesentlichen aus einem in eine Kammer oder in eine Leitung eines hydraulischen Systems einschraubbaren Körper besteht und die ebenfalls zur Messung und Überwachung des Wassergehaltes in einer Bremsflüssigkeit dient (DE-OS 3 317 638). Der einschraubbare Körper ist in diesem Fall zweckmäßigerweise als Entlüftungsschraube einer hydraulischen Anlage ausgebildet und trägt an seinem in die Flüssigkeit eintauchenden Ende als Meßsonde einen Heizdraht, der in einer Hülse mit durchbrochener Wandung angeordnet ist. Der Aussagewert der Meßergebnisse wird auch bei einem solchen einschraubbaren Körper oder Überwachungsgerät von der Funktionsfähigkeit der Meßsonde und der Signalauswertung bestimmt.

Der Erfindung liegt nun die Aufgabe zugrunde, ein Verfahren sowie eine Vorrichtung zu entwickeln, mit der sich auf vergleichsweise einfache Weise und mit relativ geringem Aufwand eine zuverlässige Aussage über die Beschaffenheit oder über den Zustand, z.B. über die Alterung, einer Druckübertragungsflüssigkeit, insbesondere eine Aussage über den Wassergehalt einer hygroskopischen Bremsflüssigkeit, gewinnen läßt. Eine entsprechende Vorrichtung sollte sich mit vergleichsweise geringem Aufwand herstellen lassen.

Es hat sich nun herausgestellt, daß diese Aufgabe in einfacher, technisch fortschrittlicher Weise durch ein Verfahren der eingangs genannten Art dadurch gelöst werden kann, daß ein Sensorelement verwendet wird, welches aufgrund seiner geometrischen Gestalt und seiner Dimensionierung nach dem Aufheizen eine Zellularkonvektion hervorruft, daß das Sensorelement mit dem konstanten Strom bis zur Einstellung einer stabilen Zellularkonvektion in einem unter der Siedetemperatur der Flüssigkeit liegenden Temperaturbereich aufgeheizt wird und daß der Spannungsabfall über dem Sensorelement als Kriterium für die Beschaffenheit der Flüssigkeit ausgewertet wird.

Bei einer Strömungsbewegung in Gasen oder Flüssigkeiten entstehen bekanntlich bei freier Konvektion (also bei einer Strömung aufgrund von Dichteunterschieden, die meist durch Temperaturunterschiede verursacht werden) unter bestimmten Umständen stabile Konvektionszellen. Solche Konvektionszellen sind stehwellenartige räumliche Strömungsgebilde mit ortsabhängiger Wärmeverteilung innerhalb des Zellraumes. Konvektionszellen stehen in geometrischer Abhängigkeit zur Körperform des Heizers und zu der zugeführten Wärmeenergie. Prinzipiell haben diese Konvektionszellen das Bestreben, etwa quadratische Querschnitte auszubilden. Es kann nur ein ganzzahliges Vielfaches derartiger Gebilde entstehen. In Abhängigkeit von der zugeführten Heizenergie ergeben sich bestimmte Konvektionszellenmuster. Eine Konvektion dieser Art wird Zellularkonvektion genannt.

Die Erfindung beruht auf der Erkenntnis, daß zum Erreichen einer Aussage über den Wassergehalt einer hygroskopischen Flüssigkeit ein Aufheizen des Sensorelementes auf die Siedetemperatur zu vermeiden ist, sondern daß durch Formgebung und Dimensionierung des Sensorelementes eine stabile Zellularkonvektion in der zu kontrollierenden Flüssigkeit bzw. im Inneren und im Bereich der Wandflächen des Sensorelementes eingestellt werden muß. Der Temperaturbereich, in dem dieses Phänomen stattfindet, liegt unterhalb der Siedetemperatur. Durch Einschalten eines rechnerisch oder empirisch ermittelten konstanten Stromes, d.h. Wechsel- oder Gleichstromes, wird während des Prüfvorganges sehr schnell der Arbeitspunkt bzw. der Arbeitsbereich der Zellularkonvektion erreicht. Eine weitere Steigerung der Heizleistung ist nicht zweckmäßig, weil in der Nähe des Siedepunktes der Flüssigkeit im Bereich des Sensorelementes eine turbulente Strömung, sogenannte Siedeturbulenzen einsetzen, der Bereich der Zellularkonvektion also verlassen wird, was schließlich, abgesehen von Ausnahmefällen, zu nicht reproduzierbaren und nicht verwertbaren Meßergebnissen führt.

Nach einer vorteilhaften Ausführungsart des erfindungsgemäßen Verfahrens wird das Sensorelement mit einem Wechselstrom konstanter Frequenz und konstanter Amplitude gespeist, wodurch, im Gegensatz zur Verwendung von Gleichstrom, galvanische Zersetzungen im Bereich des Sensorelementes vermieden werden.

Der Spannungsabfall über dem Sensorelement wird zweckmäßigerweise nach einer vorgegebenen Verzögerungszeit, die zum Aufheizen des Elementes und zur Ausbildung der stabilen Zellularkonvektion benötigt wird, gemessen. Die Änderung des elektrischen Widerstandes des Sensorelementes im Vergleich zu einer Messung in einer hochreinen Flüssigkeit der gleichen Art, läßt sich unmittelbar als Maß für die Beschaffenheit, insbesondere für den Wassergehalt der Flüssigkeit auswerten.

Eine erfindungsgemäße Vorrichtung zur Ermittlung der Beschaffenheit einer Druckübertragungsflüssigkeit und insbesondere zur Durchführung des erfindungsgemäßen Verfahrens ist in Anspruch 5 beschrieben. Weiterbildungen dieser Vorrichtung sind in den Ansprüchen 6 bis 10 definiert.

Wichtig ist, daß das Sensorelement einen Hohlraum mit durchbrochener Wandung umfaßt und derart gestaltet ist, daß nach dem Aufheizen mit Hilfe des konstanten Stromes auf einen unterhalb der Siedetemperatur der Flüssigkeit liegenden Temperaturbereich eine stabile Zellularkonvektion in dem Inneren und/oder in dem Bereich der Wandung des Sensorelementes entsteht.

Zweckmäßigerweise wird das Sensorelement in Form einer Hohlwendel, einer perforierten Röhre oder eines durch gitter- oder netzartige Wandflächen begrenzten Hohlkörpers ausgebildet. Hierzu kann gemäß einer weiteren Ausführungsart der Erfindung das Sensorelement in Form eines hohlen Quaders mit durchbrochener Wandfläche oder eines ähnlichen Stützkörpers aus Keramik oder aus einem anderen elektrisch nicht leitenden Material ausgebildet sein, wobei auf die Wandfläche mäanderförmig oder durch Aufwickeln Heizdrähte, Heizbänder oder dergleichen aufgebracht sind.

Ein besonders geringer Herstellungsaufwand ergibt sich dann, wenn die Wandflächen auf den Stützkörpern des Sensorelementes durch Ätztechnik, Feinätztechnik oder dergleichen hergestellt sind.

Weitere Merkmale, Vorteile und Anwendungsmöglichkeiten der Erfindung gehen aus der folgenden Beschreibung von Ausführungsbeispielen anhand der beigefügten Abbildungen hervor.

Es zeigen in schematisch vereinfachter Darstellung

Fig. 1 im Diagramm den prinzipiellen Widerstandsverlauf in Abhängigkeit von dem Stromfluß durch ein Sensorelement,

Fig. 2 den grundsätzlichen Aufbau einer Meßschaltung bei Durchführung des erfindungsgemäßen Verfahrens,

Fig. 3 in Teildarstellung eine Ausführungsform eines Sensorelementes nach der Erfindung,

Fig. 4 drei Varianten des Sensorelementes nach Fig. 3,

Fig. 5 in perspektivischer Darstellung als weitere Ausführungsform eines Sensorelementes nach der Erfindung einen Rahmen mit aufgespannten Heizdrähten,

Fig. 6 in perspektivischer Darstellung einen tiefen Rahmen mit aufgewickeltem Heizdraht und

Fig. 7 in perspektivischer Darstellung einen quaderförmigen Körper, dessen Wandflächen in zwei Ebenen durchbrochen sind, mit aufgewickeltem Heizdraht.

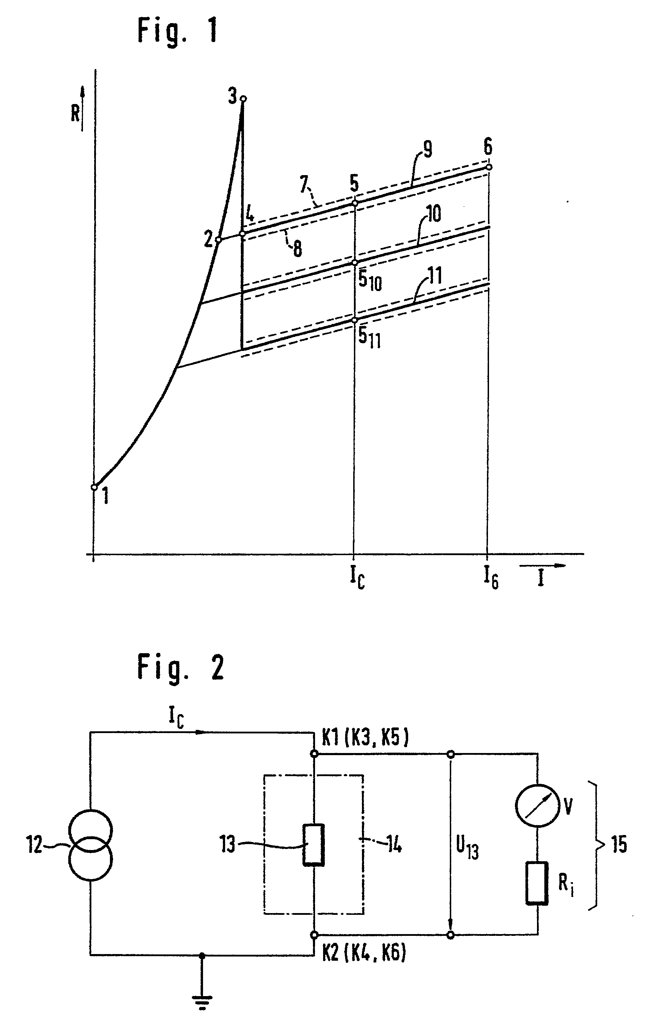

Fig. 1 veranschaulicht in vereinfachter, verallgemeinerter Form den grundsätzlichen Widerstandsverlauf in Abhängigkeit von dem Heizstrom bei Vewendung von Sensorelementen gemäß der Erfindung. Fig. 1 dient auch zur Erläuterung der erfindungsgemäßen Überlegungen und Arbeitsweise.

Bei steigendem Strom beginnt die Widerstandskurve im Arbeitspunkt "1", der folglich den Kaltleitewiderstand des Sensorelementes wiedergibt. Mit steigender Temperatur, hervorgerufen durch erhöhten Strom I, läßt sich zunächst bis zum Arbeitspunkt "3" ein überproportionales Ansteigen des Widerstandes R beobachten, der dann etwa bis zum Punkt "4" sinkt, um schließlich in Richtung der Arbeitspunkte "5" und "6" entsprechend der Kennlinie 9 annähernd konstant anzusteigen, wobei eine überlagerte, relativ geringe Schwankung innerhalb der gestrichelt dargestellten Kennlinien 7 und 8 festzustellen ist. Jenseits des Arbeitspunktes "6" ist mit vernünftigem Aufwand kein reproduzierbarer, als Meßwert auswertbarer Widerstandsverlauf in Abhängigkeit von dem Speisestrom I gegeben, weshalb hier der Strom auf den mit $I_6$ gekennzeichneten Wert beschränkt wurde.

Beim Absinken des Stromes I folgt der Widerstand stets der Kennlinie "6" – "5" – "4" – "2" – "1".

Der Kennlinienteil "1" – "2" – "3" läßt sich durch eine Parabel vom Grad n=3 annähern. Die Strecke "2" – "4" – "5" – "6" ist näherungsweise eine Gerade mit flacher Steigung, der, wie gesagt, Schwankungen mit überlagert sind.

Die Erfindung beruht auf der Erkenntnis und praktischen Verwertung der Tatsache, daß eine brauchbare Aussage über den Zustand der untersuchten Flüssigkeit bzw. über den Wassergehalt einer Bremsflüssigkeit weder in den Kennlinienbereichen "1" – "3" – "4", noch jenseits des Arbeitspunktes "6" zu erhalten ist. Eine brauchbare, einen zuverlässigen Meßwert liefernde Vorrichtung läßt sich erfindungsgemäß jedoch dann erreichen, wenn das Sensorelement durch Formgebung und Dimensionierung sowie durch Speisung mit einem konstanten Strom $I_C$ derart ausgebildet wird, daß sich der Arbeitspunkt "5" einstellt. Mit unterschiedlichem Wassergehalt verschiebt sich nämlich der Kennlinienteil "2" – "5" annähernd parallel, wie durch die Geraden 9, 10 und 11 zum Ausdruck kommt. Dabei sinkt der Widerstand mit steigendem Wassergehalt. Bei Wahl des Arbeitspunktes "5" auf der Kennlinie bzw. "5$_{10}$" und "5$_{11}$" auf den Kennlinien 10 und 11 durch entsprechende Einstellung des konstanten Stromes I$_C$ liegt der Arbeitspunkt etwa in der Mitte des linearen Kennlinienbereiches, so daß geringfügige Verschiebungen des Arbeitspunktes den Meßvorgang höchstens unerheblich beeinträchtigen.

Das Einstellen eines Arbeitspunktes in dem Kennlinienteil "4" – "6" gelingt nur dann, wenn das Sensorelement derart ausgebildet und dimensioniert ist, daß sich bei dem Strom I$_C$ eine stabile Zellularkonvektion einstellt. Wird durch höhere Energiezufuhr der Arbeitspunkt der Siedetemperatur angenähert, geht die Zellularkonvektion im Bereich des Sensorelementes in turbulente Strömung über, wodurch dann die eindeutige, als Überwachungskriterium verwertbare Abhängigkeit des Widerstandes der Meßzelle von dem Wassergehalt der Flüssigkeit endet. Das Arbeiten im Bereich stabiler Zellularkonvektion ist daher für das Erzielen eines brauchbaren Meßergebnisses von entscheidender Bedeutung.

Gemäß Fig. 2 besteht die Grundschaltung für eine Meßvorrichtung der erfindungsgemäßen Art im wesentlichen aus einer Quelle 12, die den konstanten Gleich- oder Wechselstrom I$_C$ liefert, aus dem eigentlichen Sensorelement 13, das sich im Inneren eines mit der zu untersuchenden Flüssigkeit gefüllten Gefäßes 14 befindet, sowie schließlich aus einem hochohmigen (Innenwiderstand R$_i$) Spannungsmesser 15, der an den Klemmen K1, K2 angeschlossen ist.

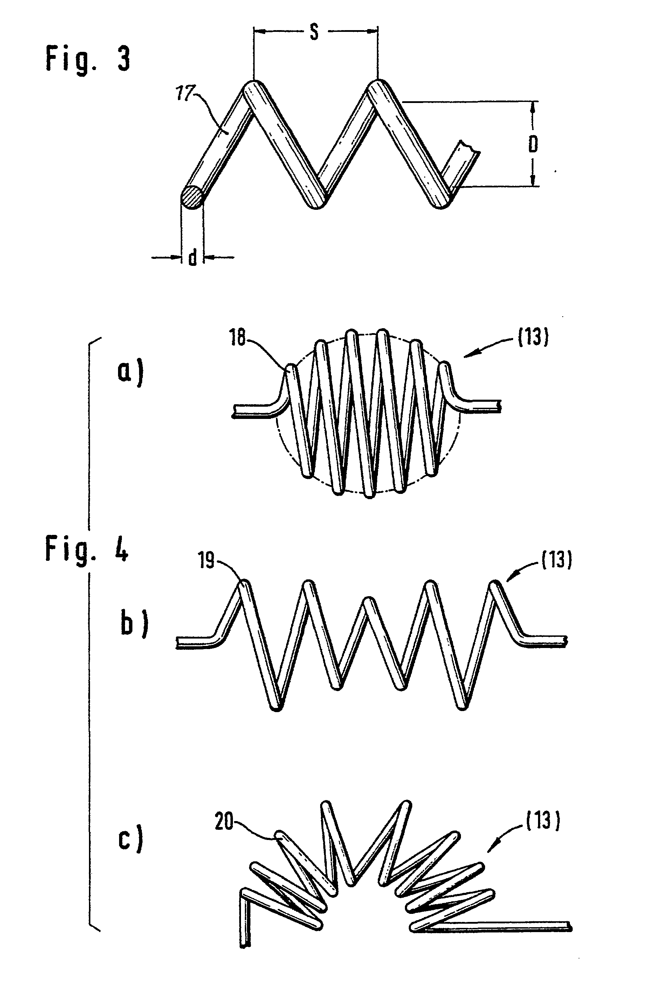

Ausführungsbeispiele des Sensorelementes 13 zeigen die Figuren 3 bis 7. Für die Raumform des Sensorelementes ist es von entscheidender Bedeutung, daß es einen Hohlraum bildet, dessen Wandungen durchbrochen sind, so daß die Flüssigkeit im Inneren des Hohlraumes mit der ihn umgebenden Flüssigkeit in Verbindung steht. Nur bei einer solchen Ausbildung kann sich eine stabile Zellularkonvektion im Inneren und/oder im Bereich der Wandungsflächen ausbilden.

Nach einem besonders einfachen Ausführungsbeispiel, das Fig 3 zeigt, besteht das Sensorelement 13 aus einer Hohlwendel, die aus einem Platin-Iridium (90% /10%) – Draht 17 gewickelt wurde. Der Draht-Durchmesser "d" betrug in diesem Fall 50 Mikrometer, der Wendeldurchmesser "D" 200 Mikrometer, die Steigung "S" 240 Mikrometer. Das Sensorelement setzte sich in diesem Beispiel aus 20 Windungen zusammen. Der Kaltwiderstand (entsprechend dem Arbeitspunkt "1" in Fig. 1) betrug 2,3 Ohm. Der Betriebsstrom I$_C$ wurde auf 700 Milliampere eingestellt. Die Hohlwendel erwies sich sowohl in horizontaler als auch in vertikaler Anordnung zur Bestimmung des Wassergehaltes einer Bremsflüssigkeit als gut geeignet.

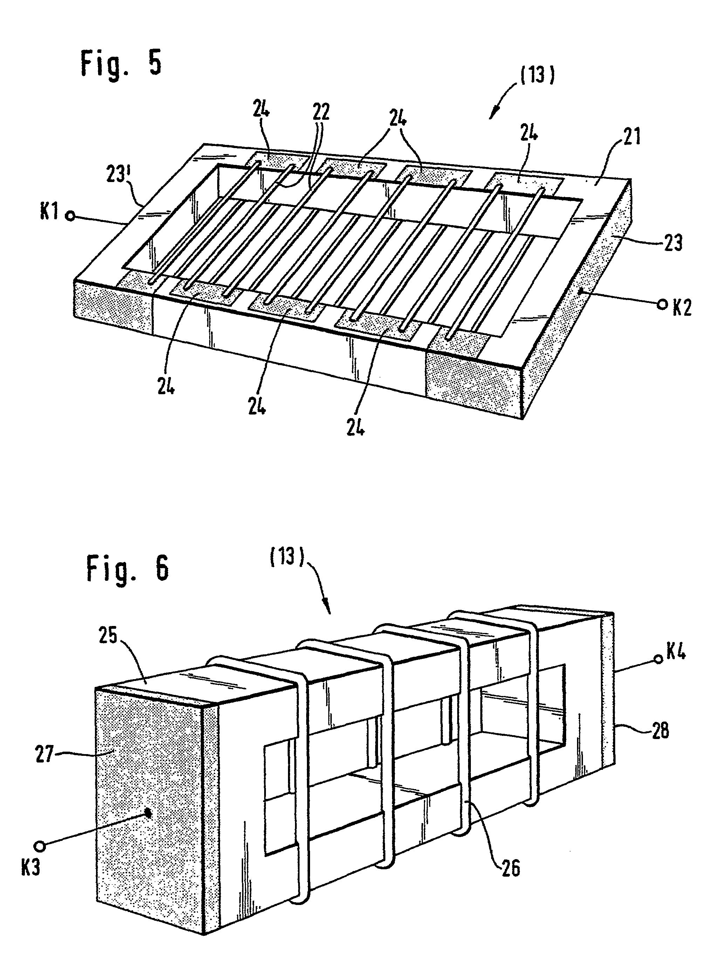

Weitere hohlwendelförmige oder hohlwendelähnliche Konfigurationen des Sensorelementes 13 zeigt Fig. 4. Die jeweilige Form wird zweckmäßigerweise rechnerisch und/oder empirisch derart festgelegt, daß sich in dem Hohlraum, den der gewandelte Draht 18, 19 oder 20 einschließt, im Bereich der Wandung und in der unmittelbaren Umgebung des Sensorelementes eine stabile Zellularkonvektion einstellt. Durch die Gestaltung der Wendel kann die Ausbildung eines bevorzugten Konvektionszellenmusters erreicht werden.

Das in Fig. 5 dargestellte Sensorelement 13 besitzt einen rahmenförmigen Stützkörper 21, der aus Keramik oder einem anderen elektrisch nicht leitenden Material besteht und als Substrat für einen mäanderförmig oder gitterförmig aufgebrachten Heizdraht 22 dient. In diesem Fall wird von der Dicke des Rahmens die Höhe des Innenraums bestimmt. Durchflutet wird das Sensorelement nach Fig. 5 in einer Ebene.

Heizdrähte 22 können, wie dargestellt, beidseitig aufgebracht werden. In speziellen Ausführungsbeispielen kann eine einseitige Ausrüstung des Rahmens mit Heizdraht 22 genügen.

Angeschlossen wird das Sensorelement nach Fig. 5 über die beiden Klemmen K1, K2 die mit einer metallischen Beschichtung 23, 23`, elektrisch leitfähig in Verbindung stehen. Die einzelnen Querstäbe des Drahtes 22 sind über metallische Kontaktbeschichtungen 24 miteinander verbunden.

Ein Sensorelement der in Fig. 5 gezeigten Art läßt sich auch in Ätztechnik oder Feinätztechnik herstellen, indem zunächst die Außenfläche des gesamten Körper metallisch beschichtet und sodann die dargestellten Leiterbahnen 22 durch Wegätzen der nicht benötigen Teile freigelegt werden.

Der Stützkörper 25 des Sensorelementes nach Fig. 6 läßt sich im Vergleich zu dem Ausführungsbeispiel nach Fig. 5 als ein tiefer Rahmen oder als ein hohler Quader, der in einer Ebene durchflossen wird, beschreiben. Auf diesem Stützkörper 25 ist ein Heizdraht 26 aufgewickelt. Über Metallkappen 27, 28 und Anschlüsse K3, K4 wird der Heizstrom oder Betriebsstrom I$_C$ zugeführt. Aus dem Spannungsabfall zwischen den Metallkappen 27, 28 und dem Strom I$_C$ läßt sich der Widerstand des dargestellten Meßelementes und damit der Wassergehalt der Bremsflüssigkeit bestimmen.

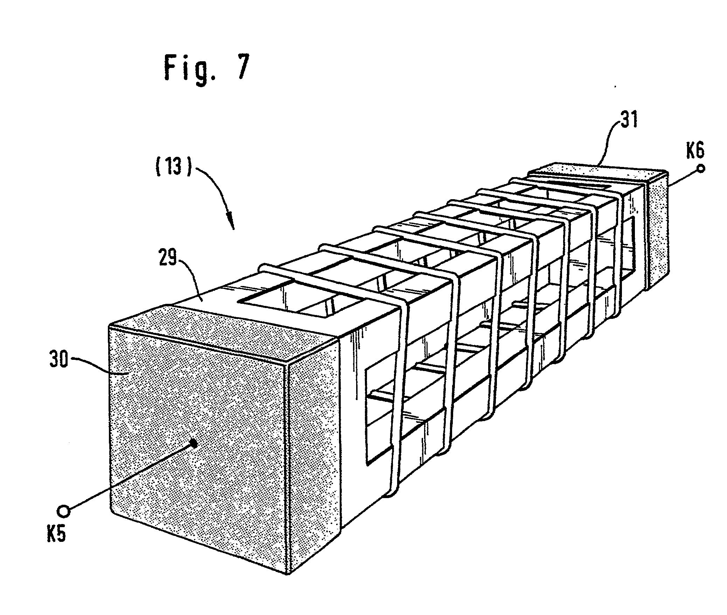

Schießlich ist in Fig. 7 noch eine weitere Ausführungsart des Sensorelementes der erfindungsgemäßen Meßvorrichtung wiedergegeben, dessen Stützkörper 29 im Gegensatz zu dem Stützkörper 25 nach Fig. 6 eine Durchflutung des Sensorelementes in zwei zueinander senkrechten Ebenen gestattet.

Die vier Seitenflächen des langgestreckten Quaders sind nämlich in diesem Fall durchbrochen. Klemmen K5, K6 zur Zuführung des konstanten Stromes I$_C$ und zum Anschluß des Voltmeters 15, vergl. Fig. 2, sind wiederum mit metallischen Kappen 30, 31 auf den Stirnflächen des Quaders verbunden.

Zahlreiche weitere Raumformen kommen für ein zur Durchführung des erfindungsgemäßen Verfahrens brauchbares Sensorelement in Frage, sofern sie die Ausbildung einer stabilen Zellularkonvektion, die bei einer unter dem Siedebereich der zu kontrollierenden Flüssigkeit liegenden Temperatur einsetzt, begünstigen.

## Patentansprüche

1. Verfahren zur Ermittlung der Beschaffenheit einer Druckübertragungsflüssigkeit, insbesondere zur Kontrolle oder Überwachung des Wassergehaltes einer hygroskopischen Bremsflüssigkeit, bei dem ein elektrisch beheizbares Sensorelement (13) in die Flüssigkeit eingetaucht und mit konstantem elektrischen Strom beschickt wird und bei dem der temperaturabhängige elektrische Widerstand des Sensorelementes (13) gemessen wird, dadurch gekennzeichnet, daß ein Sensorelement (13) vewendet wird, welches aufgrund seiner geometrischen Gestalt und seiner Dimensionierung nach dem Aufheizen eine Zellularkonvektion hervorruft, daß das Sensorelement (13) mit dem konstanten Strom bis zur Einstellung einer stabilen Zellularkonvektion in einem unter der Siedetemperatur der Flüssigkeit liegenden Temperaturbereich aufgeheizt wird und daß der Spannungsabfall ($U_{13}$) über dem Sensorelement (13) als Kriterium für die Beschaffenheit der Flüssigkeit ausgewertet wird.

2. Verfahren nach Anspruch 1, dadurch gekennzeichnet, daß der Spannungsabfall ($U_{13}$) über dem Sensorelement (13) nach einer vorgegebenen Verzögerungszeit, die zum Aufheizen und Ausbilden der stabilen Zellularkonvektion dient, gemessen und ausgewertet wird.

3. Verfahren nach Anspruch 1 oder 2, dadurch gekennzeichnet, daß die Änderung des elektrischen Widerstandes (R) des Sensorelementes (13) im Vergleich zu einer Messung in einer hochreinen Flüssigkeit gleicher Art als Maß für die Beschaffenheit, insbesondere für den Wassergehalt der Flüssigkeit, ausgewertet wird.

4. Verfahren nach einem der Ansprüche 1 bis 3, dadurch gekennzeichnet, daß das Sensorelement (13) mit Wechselstrom konstanter Frequenz und konstanter Amplitude gespeist wird.

5. Vorrichtung zur Ermittlung der Beschaffenheit einer Druckübertragungsflüssigkeit, insbesondere zur Kontrolle oder Überwachung des Wassergehaltes einer hygroskopischen Bremsflüssigkeit, mit einem elektrisch beheizbaren Sensorelement (13), das einen Hohlraum aufweist und das in die zu untersuchende Flüssigkeit eintaucht, mit einer Stromquelle (12), die einen konstanten elektrischen Strom liefert, und mit einer Meßeinrichtung (15), mit der der Spannungsabfall über dem Sensorelement (13) beim Einspeisen des konstanten elektrischen Stromes abgreifbar und der temperaturabhängige elektrische Widerstand des Sensorelementes (13) ermittelbar ist, dadurch gekennzeichnet, daß der Hohlraum des Sensorelementes (13) eine durchbrochene Wandung aufweist und daß das Sensorelement (13) derart gestaltet sowie dimensioniert ist, daß nach dem Aufheizen mit Hilfe des konstanten Stromes eine stabile Zellularkonvektion in einem unterhalb der Siedetemperatur der Flüssigkeit liegenden Temperaturbereich entsteht, und daß die Meßeinrichtung (15) derart gestaltet ist, daß sie den bei der stabilen Zellularkonvektion an dem Sensorelement (13) auftretenden Spannungsabfall als Kriterium für die Beschaffenheit der Flüssigkeit auswertet.

6. Vorrichtung nach Anspruch 5, dadurch gekennzeichet, daß das Sensorelement (13) in Form einer Hohlwendel, einer perforierten Röhre oder eines durch gitter- oder netzförmige Randflächen begrenzten Hohlkörpers ausgebildet ist.

7. Vorrichtung nach Anspruch 5 oder 6, dadurch gekennzeichnet, daß das Sensorelement (13) in Form eines rechteckigen Rahmens, eines hohlen Quaders mit durchbrochener Randfläche oder eines ähnlichen Stützkörpers ausgebildet ist, der aus Keramik oder aus einem anderen elektrisch nicht leitenden Material besteht und dessen Wandflächen zumindest teilweise durch mäanderförmig angeordnete oder aufgewickelte Heizdrähte (17–20, 22, 26), Heizbänder oder dergleichen gebildet sind.

8. Vorrichtung nach einem der Ansprüche 5 bis 7, dadurch gekennzeichnet, daß die Wandflächen des Sensorelementes (13) durch Ätztechnik hergestellt sind.

9. Vorrichtung nach Anspruch 7 oder 8, dadurch gekennzeichnet, daß der Heizdraht (17–20, 22, 26) oder das Heizband des Sensorelementes (13) aus einer Platin-Iridium oder einer Platin-Rhodium-Legierung, aus Wolfram oder dergl. besteht.

10. Vorrichtung nach einem der Ansprüche 5 bis 9, dadurch gekennzeichnet, daß als Sensorelement (13) eine Hohlwendel vorgesehen ist, die aus einem Platin-Iridium-Draht besteht und einen Drahtdurchmesser (d) von 30 bis 60 Mikrometer, einen Kerndurchmesser (D) von 100 bis 200 Mikrometer, eine Wendelsteigung (S) von 200 bis 300 Mikrometer, einen Kaltwiderstand von ca. 2 bis 4 Ohm aufweist und eine Windungzahl zwischen 15 und 25 besitzt.

## Claims

1. A method of determining the condition of a pressure transmitting fluid, especially for checking or monitoring the water content of a hygroscopic brake fluid, wherein an electrically heatable sensor element (13) is immersed into the fluid and to which a constant electric current is applied, and wherein the temperature-responsive electrical resistance of the sensor element (13) is measured, characterized in that a sensor element (13) is used which is geometrically configured and dimensioned such that, after heating of the element, a cellular convection arises, in that the sensor element (13) is heated with the constant current until attainment of a stable cellular convection in a range of temperature below the boiling temperature of the fluid, and in that the voltage drop ($U_{13}$) on the sensor element (13) is analysed as a criterion for the condition of the fluid.

2. A method as claimed in claim 1, characterized in that the voltage drop ($U_{13}$) on the sensor element (13) after a predetermined delay time serving for heating and forming the stable cellular convection is measured and analysed.

3. A method as claimed in claim 1 or 2, characterized in that the change in the electrical resistance (R) of the sensor element (13), as compared with a measurement in a high-purity fluid of this type, is taken as a standard of the condition, especially of the water content, of the fluid.

4. A method as claimed in any one of the claims 1 to 3, characterized in that the sensor element (13) is energized by alternating current of a constant frequency and constant amplitude.

5. An apparatus for determining the condition of a pressure transmitting fluid, especially for checking or monitoring the water content of a hygroscopic brake fluid, comprising an electrically heatable sensor element (13) having a cavity and being immersed into the fluid to be tested, with a source of current (12) supplying a constant electric current, and with a measuring device (15) allowing to pick up the voltage drop on the sensor element (13) upon the delivery of the constant electric current and to determine the temperature-responsive electrical resistance of the sensor element (13), characterized in that the cavity of the sensor element (13) has a perforated wall, and in that the sensor element (13) is of a configuration and dimension such that, after heating with the aid of the constant current, a stable cellular convection arises in a temperature range below the boiling temperature of the fluid, and in that the measuring device (15) is devised such as to assess the voltage drop occurring on the sensor element (13) in the event of the stable cellular convection as a criterion for the condition of the fluid.

6. An apparatus as claimed in claim 5, characterized in that the sensor element (13) is in the form of a hollow helix, a perforated tube or a hollow body confined by grid-type or reticular peripheral faces.

7. An apparatus as claimed in claim 5 or 6, characterized in that the sensor element (13) is in the form of a rectangular frame, a hollow block having a perforated peripheral face or a similar supporting body made of ceramics or any other electrically nonconductive material and the wall faces of which at least in part are formed of heating filaments (17 to 20, 22, 26), strip heaters or the like disposed in meander-type manner or wound up.

8. An apparatus as claimed in any one of the claims 5 to 7, characterized in that the wall faces of the sensor element (13) are manufactured by etching technique.

9. An apparatus as claimed in claim 7 or 8, characterized in that the heating filament (17 to 20, 22, 26) or the strip heater of the sensor element (13) is made of a platinum/iridium or a platinum/rhodium alloy, of tungsten or the like.

10. An apparatus as claimed in anyone of the claims 5 to 9, characterized in that the sensor element (13) is a hollow helix composed of a platinum/iridium wire and being of a wire diameter (d) of between 30 and 60 micrometers, a core diameter (D) of between 100 and 200 micrometers, a helix pitch (S) of between 200 and 300 micrometers, a thermoresistance of about 2 to 4 ohm and a number of turns of between 15 and 25.

**Revendications**

1. Procédé pour déterminer la condition d'un liquide de transmission de pression, en particulier pour contrôler ou surveiller la teneur en eau d'un liquide de freinage hygroscopique, dans lequel un élément de capteur à chauffage électrique (13) est immergé dans le liquide et alimenté en courant électrique constant, et dans lequel la résistance électrique de l'élément de capteur (13) est mesurée en fonction de la température, caractérisé en ce qu'un élément de capteur (13) est utilisé, qui, en raison de sa forme géométrique et de ses dimensions, provoque une convection cellulaire à l'issue du chauffage, en ce que l'élément de capteur (13) est chauffé avec le courant constant jusqu'à l'obtention d'une convection cellulaire stable dans une plage de température inférieure à la température d'ébullition du liquide, et en ce que la chute de tension ($U_{13}$) au niveau de l'élément de capteur (13) est exploitée comme un critère de la condition du liquide.

2. Procédé conforme à la revendication 1, caractérisé en ce que la chute de tension ($U_{13}$) au passage de l'élément de capteur (13) est mesurée et exploitée après un temps de retard prédéfini qui est nécessaire pour chauffer l'élément et pour former la convection cellulaire stable.

3. Procédé conforme à la revendication 1 ou 2, caractérisé en ce que la variation de la résistance électrique (R) de l'élément de capteur (13) par rapport à une mesure effectuée dans un liquide de grande pureté de type analogue est directement exploitée comme la mesure de la condition, en particulier de la teneur en eau du liquide.

4. Procédé conforme à l'une quelconque des revendications 1 à 3, caractérisé en ce que l'élément de capteur (13) est alimenté avec du courant alternatif de fréquence et d'amplitude constantes.

5. Dispositif pour déterminer la condition d'un liquide de transmission de pression, en particulier pour contrôler ou surveiller la teneur en eau d'un liquide de freinage hygroscopique, comprenant un élément de capteur (13) pouvant être chauffé électriquement, qui comporte une cavité et qui plonge dans le liquide à analyser, une source de courant (12) qui fournit un courant électrique constant, et un dispositif de mesure (15) qui permet de connaître la chute de tension au passage de l'élément de capteur (13) lors de l'alimentation en courant électrique constant et de déterminer la résistance électrique de l'élément de capteur (13) en fonction de la température, caractérisé en ce que la cavité de l'élément de capteur (13) comporte une paroi percée, en ce que l'élément de capteur (13) est conçu et dimensionné de façon qu'après avoir été chauffé à l'aide du courant constant, il se produit une convection cellulaire stable dans une plage de température inférieure à la température d'ébullition du liquide, et en ce que le dispositif de mesure (15) est conçu de façon que la chute de tension de produisant su niveau de l'élément de capteur (13) lors de la convection cellulaire stable est exploitée comme un critère de la condition du liquide.

6. Dispositif conforme à la revendication 5, caractérisé en ce que l'élément de capteur (13) est conçu sous la forme d'une spirale creuse, d'un tube perforé ou d'un corps creux limité par des surfaces de paroi en forme de treillis ou de filet.

7. Dispositif conforme à l'une quelconque des revendications 5 ou 6, caractérisé en ce que l'élément de capteur (13) est conçu sous la forme d'un cadre rectangulaire, d'un parallélépipède creux à surface de paroi percée ou d'un corps d'appui analogue réalisé en céramique ou dans un autre matériau électrique non conducteur, dont les surfaces de paroi sont formées au moins partiellement par des fils chauffants (17 à 20, 22, 26), des bandes chauffantes ou des éléments analogues.

8. Dispositif conforme à l'une quelconque des revendications 5 à 7, caractérisé en ce que les surfaces de paroi de l'élément de capteur (13) sont réalisées par attaque chimique.

9. Dispositif conforme à l'une quelconque des revendications 7 ou 8, caractérisé en ce que le fil chauffant (17, 20, 22, 26) ou la bande chauffante de l'élément de capteur (13) sont réalisés en alliage de platine irridié ou rhodié, en tungstène ou dans une autre matière.

10. Dispositif conforme à l'une quelconque des revendications 5 à 9, caractérisé en ce que l'élément de capteur (13) est constitué d'une spirale creuse réalisée à partir d'un fil en platine irridié et possédant un diamètre de fil (d) de 30 à 60 microns, un diamètre de noyau (D) de 100 à 200 microns, un pas de spirale (S) de 200 à 300 microns, une résistance à froid de 2 à 4 ohms et un nombre de spires compris entre 15 et 25.

# Fig. 1

# Fig. 2

Fig. 3

17

S

D

d

18 (13)

a)

Fig. 4

19 (13)

b)

20 (13)

c)

EP 0 208 096 B1

Fig. 5

Fig. 6

# Fig. 7